## Europäisches Patentamt
## European Patent Office
## Office européen des brevets

(19)

(11) Numéro de publication: **0 324 665**
**A1**

(12)

# DEMANDE DE BREVET EUROPEEN

(21) Numéro de dépôt: **89400021.5**

(22) Date de dépôt: **04.01.89**

(51) Int. Cl.⁴: **C 01 F 17/00**

(30) Priorité: **14.01.88 US 143978**

(43) Date de publication de la demande:
**19.07.89 Bulletin 89/29**

(84) Etats contractants désignés:
**AT BE CH DE FR GB IT LI NL SE**

(71) Demandeur: **RHONE-POULENC CHIMIE**
**25, quai Paul Doumer**
**F-92408 Courbevoie Cédex (FR)**

(72) Inventeur: **Gradeff, Peter S.**
**Black River Road**
**Pottersville New Jersey 07979 (US)**

**Schreiber, Fred G.**
**100 Lawrence Avenue**
**Highland Park New Jersey 08904 (US)**

(74) Mandataire: **Dutruc-Rosset, Marie-Claude et al**
**RHONE-POULENC INTERSERVICES Service Brevets**
**Chimie 25, Quai Paul Doumer**
**F-92408 Courbevoie Cédex (FR)**

(54) **Complexe nitrate céreux-nitrate d'ammonium anhydre et procédé pour sa fabrication à partir de nitrate cérique d'ammonium.**

(57) L'invention a pour objet un procédé pour la fabrication d'un complexe de nitrate céreux et de nitrate d'ammonium anhydre.

Le procédé selon l'invention comprend les étapes suivantes :

(a) on met en contact le nitrate cérique d'ammonium exempt d'eau avec un agent réducteur convenable ; et

(b) on maintient en contact pendant une durée suffisante pour réduire le cérium (IV) en cérium (III) et former le complexe de nitrate céreux anhydre.

Application : synthèses d'autres dérivés de Ce(III) exigeant un produit de départ anhydre.

EP 0 324 665 A1

## Description

**Complexe nitrate céreux-nitrate d'ammonium anhydre et procédé pour sa fabrication à partir de nitrate cérique d'ammonium.**

La présente invention a pour objet un complexe nitrate céreux-nitrate d'ammonium anhydre et son procédé de fabrication.

Les techniques modernes de séparation des lanthanides donnent ces éléments principalement sous forme de chlorures ou de nitrates en solution aqueuse. Ils sont utilisés pour fabriquer d'autres dérivés tels que les carbonates, hydroxydes, oxydes, etc. Ils peuvent aussi être utilisés pour fabriquer certains des dérivés organiques tels que les alkylcarboxylates supérieurs ou les acétylacétonates. Cependant, les sels inorganiques de lanthanides anhydres sont essentiels pour fabriquer les alcoolates les dérivés ayant des liaisons Ln-carbone et de nombreux des autres, ainsi que pour produire des métaux par des procédés électrolytiques et métallothermiques. Les oxydes peuvent être fabriqués à l'état anhydre, mais leur faible solubilité et leur réactivité limitée excluent leur utilisation dans de nombreux domaines. Les halogénures sont la seule classe de composés qui sont utilisés comme source de composés anhydres convenant à cet effet. Les plus faciles à fabriquer sont les fluorures. Cependant, ils n'ont qu'une utilisation limitée dans les synthèses en raison d'une solubilité et d'une réactivité extrêmement faibles. Les plus difficiles à déshydrater sont les iodures. Les bromures et les chlorures présentent un degré semblable de difficulté qui est moins grand que pour les iodures, mais plus grand que pour les fluorures. Au vu de considérations économiques et d'environnement, les chlorures sont les sels de lanthanides anhydres les plus recherchés. Ceci comprend le cérium, qui est le plus abondant des lanthanides.

Les halogénures qui se séparent de leurs solutions aqueuses retiennent ordinairement 6 à 7 moles d'eau. Après séparation de l'eau non liée, la majeure partie de l'eau liée peut être séparée par déshydratation soignée au-dessous de 100°C. Il est cependant extrêmement difficile de séparer la dernière mole d'eau sans décomposer l'halogénure.

La déshydratation des hydrates de chlorure de lanthane peut être effectuée par HCl gazeux, mais c'est un procédé fastidieux. La déshydratation utilisant le chlorure d'ammonium en plus d'HCl et au-dessous de 200-300°C a donné les meilleurs résultats. On a fait réagir des oxydes avec le monochlorure de soufre et le chlore ou le monochlorure de soufre seul et le chlorure de thionyle, le tétrachlorure de carbone et le phosgène ont également été utilisés comme réactifs. La préparation des sels de lanthanides anhydres les plus couramment utilisés, les chlorures, n'est pas facile (Chem. Rev. 1962, pages 503-511).

Le brevet des EUA n° 4 492 655 aux noms de Gradeff et Schreiber décrit la préparation de dérivés cyclopentadiénylés du cérium (III) utilisant le nitrate cérique d'ammonium et le cyclopentadiényl-Na. Par utilisation d'un nitrate, on s'est écarté des voies classiques. Le nitrate cérique d'ammonium avait l'avantage d'être facilement obtenu sous forme anhydre. Le procédé de cette invention consiste à ajouter lentement le cyclopentadiénylure de métal alcalin à une solution de nitrate cérique d'ammonium pour former successivement du mono jusqu'au tricyclopentadiénylcérium. L'équation globale de la réaction est la suivante

$$2[Ce(NO_3)_4.2NH_4NO_3] + 12NaCp \rightarrow 2Ce(Cp)_3 + 4CpH + (Cp)_2 + 12NaNO_3 + 4NH_3$$

On montre que l'étape de réduction "in situ" se déroule par deux voies possibles.

$$Ce(NO_3)_4 + 4NaCp \rightarrow Ce(Cp)_4 + 4NaNO_3$$
$$2Ce(Cp)_4 \rightarrow 2Ce(Cp)_3 + Cp_2$$

La réaction "in situ" ci-dessus est intéressante dans les cas où le réactif est un bon agent réducteur, une partie du réactif étant consommée dans la réduction de $Ce^{+4}$ en $Ce^{+3}$. Dans les cas comme celui indiqué ci-dessus, ceci peut être assez coûteux.

La présente invention a pour objet de proposer un composé anhydre de cérium (III) qui peut être utilisé pour les synthèses d'autres dérivés souhaitables de $Ce^{+3}$ qui exigent un produit de départ anhydre. Le but est de préparer le complexe anhydre $Ce(NO_3)_3.3NH_4NO_3$. Un autre but de la présente invention est de proposer un procédé convenable pour fabriquer ce complexe, qui évite la nécessité de températures élevées et de réactifs difficiles à mettre en oeuvre rencontrée normalement dans la préparation des halogénures anhydres.

Jusqu'à la présente invention, la déshydratation des hydrates de nitrates céreux selon des procédés connus dans la technique ne donnait pas de produit anhydre (Russian Journal of Inorganic Chemistry, novembre 1965, pages 1373-1377).

La présente invention a pour objet un procédé approprié pour fabriquer le nitrate céreux anhydre. Il consiste à traiter le complexe hexanitratocérate (IV) d'ammonium anhydre, également dénommé nitrate cérique d'ammonium, par un agent réducteur (ra) convenable, $Ce^{+4}$ étant réduit en $Ce^{+3}$ en conditions anhydres. Le composé de $Ce^{+3}$ résultant, un complexe de nitrate de $Ce^{+3}$ et de nitrate d'ammonium, peut être utilisé comme source de sels anhydres de $Ce^{+3}$.

L'invention peut être représentée par l'équation A :

A. $(NH_4)_2Ce(NO_3)_6 + (ra) \rightarrow Ce(NO_3)_3.2NH_4NO_3 + HNO_3 + (PrOX)$

dans laquelle (PrOX) désigne le produit d'oxydation qui dépend de la nature de (ra).

Le nitrate cérique d'ammonium (CAN) est un produit du commerce qui peut être débarrassé de toute l'eau par simple séchage dans un four à 105-120°C et sous pression atmosphérique pendant 6-12 h ou sous vide à température inférieure. Selon la présente invention, le CAN séché soit à l'état solide, soit en suspension ou dissolution dans un solvant inerte convenable, est mis en contact avec l'agent réducteur.

Dans un mode de mise en oeuvre préféré de l'invention, on choisit l'ammoniac comme étant l'agent réducteur le plus approprié parce qu'il est économique et donne un sous-produit simple, l'azote, en plus du nitrate d'ammonium.

On pense que la réaction suivante a lieu

B.    $3(NH_4)_2Ce(NO_3)_6 + 4NH_3 \rightarrow 3Ce(NO_3)_3 + 9NH_4NO_3 + 1/2 N_2$

Le traitement du CAN solide, préalablement séché, par $NH_3$ peut s'effectuer de diverses manières. Le CAN est maintenu en contact avec $NH_3$ pendant une durée suffisante pour réduire tout le $Ce^{+4}$ en $Ce^{+3}$. La durée nécessaire à cet effet dépend du mode de contact, de la granulométrie du CAN, de la température et de la pression. La mise en contact du CAN avec $NH_3$ peut s'effectuer sous pression atmosphérique, par exemple en agitant le CAN en atmosphère de $NH_3$. L'ammoniac peut aussi être maintenu sous pression pouvant atteindre 10 atmosphères, voire même davantage, ou utilisé à l'état liquide. Lorsque l'on utilise $NH_3$ liquide, l'excès est facilement récupéré après la fin de la réaction.

La quantité d'ammoniac doit être au moins la quantité stoechiométrique, mais on utilise normalement des quantités plus grandes. L'excès d'ammoniac peut être récupéré. De faibles quantités (environ 2 moles) d'ammoniac qui restent liées par coordination au complexe de nitrate de cérium peuvent être éliminées finalement, si on le désire. La température pendant le contact peut être aussi faible que la température de l'ammoniac liquide ou aussi élevée qu'environ 130°C, mais inférieure à la température de décomposition du CAN qui commence à environ 170°C.

Le CAN peut être réduit en suspension ou en solution dans des solvants inertes tels que benzène, pentane, éther de pétrole ou éthers méthyliques de glycols (notamment le diglyme). Il n'est pas nécessaire d'isoler le produit du solvant inerte. Une fois que la réduction complète par $NH_3$ a été effectuée, on peut ajouter des réactifs pour effectuer la conversion subséquente du nitrate complexe de $Ce^{+3}$ anhydre en le dérivé de $Ce^{+3}$ désiré.

Bien que la réduction du CAN par $NH_3$ soit le moyen le plus efficace et le moins coûteux pour produire le nitrate complexe de $Ce^{+3}$ désiré, on peut aussi utiliser une foule d'autres agents réducteurs. Les plus intéressants, à cause de leur bas prix, de leur facilité relative à effectuer la réaction et de leur facilité relative à séparer le (PrOX) si nécessaire, sont les alcanols inférieurs ayant de 1 à 5 atomes de carbone tels que, par exemple, méthanol, éthanol, propanol, isopropanol, etc. En général, l'agent réducteur convenable pour la présente invention est un composé qui réagit comme réducteur en milieu anhydre et conduit à une réduction pratiquement complète de $Ce^{+4}$ en $Ce^{+3}$. Le nombre de composés qui peuvent être utilisés à cet effet est bien sûr très grand. On peut l'apprécier en citant le cas du diméthoxyéthane, considéré comme un solvant inerte, que l'on peut faire réagir avec le CAN à la température de reflux pour réduire totalement $Ce^{+4}$ en $Ce^{+3}$. La réduction peut se dérouler rapidement ou lentement selon l'agent réducteur. On peut l'accélérer si on le désire par les UV, par chauffage ou par addition d'un catalyseur convenable, par exemple, un catalyseur à base d'un métal noble déposé sur carbone. Pendant cette étape du procédé, le $Ce^{+4}$ est réduit en $Ce^{+3}$, tandis que le milieu reste anhydre. Comme indiqué dans l'équation A, de l'acide nitrique est produit pendant la réaction et reste tel que dans le mélange si l'agent réducteur est un composé neutre comme un alcool. Outre l'acide nitrique, le mélange peut aussi contenir un sous-produit d'oxydation (PrOX).

La quantité de (ra) doit être au moins la quantité stoechiométrique mais dans la pratique, on utilise un excès pour accélérer le processus de réduction. L'acide nitrique peut être neutralisé et le (PrOX) séparé du système dans le cas où le nitrate complexe de $Ce^{+3}$ doit être isolé.

Conformément au mode de réalisation préféré de l'invention qui consiste à effectuer la réduction du CAN par l'ammoniac, on obtient un complexe nitrate céreux-nitrate d'ammonium-ammoniac.

Ce complexe peut être représenté par la formule suivante (I)

$Ce(NO_3)_6, 3NH_4NO_3, xNH_3$    (I)

dans ladite formule, x est le nombre de moles d'ammoniac par mole de complexe. x est un nombre qui peut varier en fonction de séchage du produit obtenu. L'ammoniac étant utilisé, en excès, x est généralement supérieur à 0 et inférieur ou égal à 3. Cette borne maximale ne présente aucun caractère critique et peut être dépassée. Le plus souvent, x est compris entre 1 et 3.

Les tentatives pour isoler $Ce(NO_3)_3$ à l'état libre n'ont pas réussi. Apparemment, l'ion $Ce^{+3}$ doit être lié encore par coordination. En l'absence d'eau ou d'autres agents de coordination, ceci est effectué par le nitrate d'ammonium. Comme dans le cas du CAN et d'autres exemples courants pour les éléments lanthanides, les ligands de coordination peuvent être séparés, si on le désire, dans des réactions ultérieures et ne posent pas de problème important. Une certaine quantité de $NH_3$ est également liée dans la molécule au nitrate complexe de cérium et dans certains cas sa présence peut être souhaitable. Par exemple, lorsque le produit anhydre de $Ce^{+3}$ résultant doit réagir avec un alcool ou un silanol en présence de $NH_3$, le $NH_3$ lié par coordination constitue une portion du $NH_3$ nécessaire. Dans d'autres cas, sa présence peut ne pas être souhaitable et des étapes doivent être mises en oeuvre pour le séparer, par exemple pompage sous vide, chauffage, neutralisation par HCl sec ou déplacement par un autre agent de coordination.

Les exemples suivants illustrent l'invention sans toutefois en limiter la portée.

La présence ou l'absence de $Ce^{+4}$ est déterminée en ajoutant du sulfate de baryum-diphénylamine à un échantillon acidifié par l'acide acétique glacial. En présence de $Ce^{+4}$, la solution présente une couleur pourpre intense.

Exemple 1

Réduction du CAN par l'ammoniac dans le pentane

On met en suspension 70,5 g (0,128 mole) de CAN préalablement séché (jaune orangé) dans 500 ml de pentane. On remplace l'atmosphère de gaz inerte par de l'ammoniac et on agite bien tout le système pendant 24 h. Pendant cette durée, la matière en réaction devient incolore, ce qui indique que la réduction est terminée. On filtre la suspension et on sèche le nouveau composé sous vide poussé et on l'utilise sans autre purification pour les réactions ultérieures. Rendement : 79,4 g d'une poudre incolore. Le produit contient de l'ammoniac coordiné.

### Exemple 2

Réduction du CAN par l'ammoniac dans le diméthoxyéthane

On dissout 5 g (9,21 mmole) de CAN dans 60 ml de DME, on dégaze la solution, on la sature par l'ammoniac et on agite pendant une nuit. Le matin suivant, le solide en suspension a complètement perdu sa couleur jaune orangé.

### Exemple 3

Réduction du CAN par l'ammoniac, sans solvant

On agite énergiquement 5 g (9,21 mmole) de CAN sans solvant en atmosphère d'ammoniac pendant une semaine. Après cette durée, tout le CAN s'est décoloré. Le test du cérium (IV) est négatif.

### Exemple 4

On chauffe au reflux pendant une nuit 3,44 g de CAN avec 46,4 g de DME. Le jour suivant, il ne reste pas de $Ce^{+4}$ dans le ballon. Le pH résultant est de 4,0, ce qui indique que le $HNO_3$ mis en liberté a réagi avec les sous-produits d'oxydation. Il y a également des vapeurs nitreuses.

### Exemple 5

On mélange 5,0 g de CAN avec 50 ml de DME en présence de 0,4 g de catalyseur au charbon de bois. Tout le $Ce^{+4}$ est réduit en moins de 1,5 h et le pH final est de 1,0.

### Exemple 6

On mélange 5,0 g de CAN avec 45,6 g de méthanol à la température ambiante en atmosphère d'argon en agitant en présence de 0,06 g de catalyseur platine/carbone. La réduction complète est terminée en 30 min et le pH final est de 1,0.

### Exemple 7

On agite 5,0 g de CAN, 45 g d'alcool isopropylique et 0,06 g de catalyseur à 5% de ruthénium sur carbone à la température ambiante en atmosphère d'argon. La réduction complète de $Ce^{+4}$ est observée en 2 h.

### Exemple 8

On expose à la lumière solaire pendant 1 jour 2,0 g de CAN mélangé avec 20 ml de méthanol. La réduction est complète.

### Exemple 9

On répète le mode opératoire décrit à l'exemple 8, sauf que l'on remplace le méthanol par l'isopropanol, et on obtient des résultats semblables.

### Exemple 10

On répète le mode opératoire décrit à l'exemple 8, sauf que l'on remplace la lumière solaire par la lumière UV, et on obtient des résultats semblables.

### Exemple 11

On mélange 5,0 g de CAN, 44,7 g de n-butanol et 0,06 g de catalyseur à 5% de ruthénium sur carbone. La réduction complète est obtenue en 1 h 1/2.

### Exemple 12

Tentatives pour isoler le $Ce(NO_3)_3$ non complexé

L'extraction fractionnée par le méthanol dans lequel $NH_4NO_3$ est suffisamment soluble a échoué. Le $NH_4NO_3$ semble fortement lié au $Ce(NO_3)_3$. Cependant, l'ammoniac lié est complètement dissocié du complexe pendant le traitement qui laisse un complexe $CE(NO_3)_3.3NH_4NO_3$ pur.

On arrive aux mêmes conclusions après l'échec d'une tentative pour séparer $NH_4NO_3$ par sublimation sous vide poussé.

### Exemple 13

## Réaction du nitrate de cérium (III) avec l'isopropylate de sodium

A une suspension fraîchement préparée de 1,85 mmole de complexe de nitrate de cérium (III) dans le DME, on ajoute 4,17 ml (11,1 mmole) d'une solution 2,66 molaire de NaOiPr dans l'isopropanol. Après 2 h, on filtre le mélange de réaction, qui donne 801 mg de $NaNO_3$ = 87,3 % de la quantité attendue de $NaNO_3$ (il reste un peu de solide sur les parois du verre fritté). On sèche le filtrat, on le délaie dans le pentane et on filtre. Rendement : 810 mg (1,63 mmole) = 88,1 % pour $Ce(OiPr)_3(HOiPr)_3$ (PM = 497,67).

Le spectre de RMN est pratiquement identique à celui d'un composé préparé indépendamment par réaction de $CeCl_3$ avec NaOiPr dans THF/IPA.

## Exemple 14

## Préparation des méthylates et isopropylates de Ce III

On dissout le CAN préalablement séché dans un excès de méthanol et on l'expose à la lumière solaire jusqu'à ce que tout le $Ce^{+4}$ soit réduit.

On traite une portion du mélange de réaction par le gaz $NH_3$, ce qui précipite $Ce(OCH_3)_3$. On sépare le méthylate du nitrate d'ammonium par extraction et lavage au méthanol, ou bien le mélange de réaction est utilisé sans séparation dans la synthèse ultérieure.

On traite une autre portion en séparant sous vide tout l'excès de méthanol et l'acétaldéhyde qui est un produit d'oxydation du méthanol par le CAN. On mélange le solide restant avec l'isopropanol et ensuite on fait réagir avec l'isopropylate de sodium pour convertir le $Ce(NO_3)_3$ en $Ce(OiPr)_3$.

## Exemple 15

## Réaction du complexe de nitrate céreux avec le triphénylsilanol

$$Ce(NO_3)_3.3NH_4NO_3 + 3Ph_3SiOH + 3NH_3 \rightarrow Ce(OSiPh_3)_3 + 6NH_4NO_3$$

On met en suspension 5 g (8,8 mmole) de $Ce(NO_3)_3.3NH_4NO_3$ dans 40 ml (35 g) de DME. On ajoute 7,32 g (26 mmole) de $Ph_3SiOH$ à l'état solide et on agite pendant 5 min. Pendant les 5 min suivantes, on fait barboter du gaz $NH_3$ dans la suspension, avec formation d'une phase supérieure limpide jaune clair. On continue l'addition de $NH_3$ pendant les 10 min suivantes. La filtration ultérieure du mélange sur un verre fritté de Schlenk donne un filtrat jaune clair limpide. Après séparation du solvant sous le vide de la pompe à huile, on obtient 5,8 g d'une poudre blanche bien soluble dans $CHCl_3$, le DME et le THF.
Spectre de RMN de $^1H$ ($CHCl_3$-d), δ : 7,24 ; 7,31 ; 7,36 ; 7,49 ; 7,52 ; 7,57 ; 7,59.
Spectre de RMN de $^{13}C$ ($CHCl_3$-d), δ : 127,76 ; 129,93 ; 134,91.

## Exemple 16

## Dérivés alcanolatoamines de cérium (III)

On combine dans diverses proportions des portions de complexe $Ce(NO_3)_3,3NH_4NO_3$ agitées dans le méthanol avec les alcanolamines suivantes :
monoéthanolamine (1 : 1 ; 1 : 2 ; 1 : 3)
diéthanolamine (1 : 1 ; 1 : 2)
triéthanolamine (1 : 1)
diéthanolamine (1 : 1 ; 1 : 2 ; 1 : 3)

Après l'addition d'ammoniac, on obtient les alcanolatoamines correspondantes, par échange avec le $Ce(OCH_3)_3$ produit in situ.

## Revendications

1. Procédé pour la fabrication d'un complexe de nitrate céreux anhydre, caractérisé par les étapes suivantes :

   (a) on met en contact le nitrate cérique d'ammonium exempt d'eau avec un agent réducteur convenable ; et

   (b) on maintient en contact pendant une durée suffisante pour réduire le cérium (IV) en cérium (III) et former le complexe de nitrate céreux anhydre.

2. Procédé selon la revendication 1, caractérisé en ce que l'agent réducteur est le gaz ammoniac.

3. Procédé selon la revendication 2, caractérisé en ce que le nitrate cérique d'ammonium est à l'état solide.

4. Procédé selon la revendication 2, caractérisé en ce que le nitrate cérique d'ammonium est en suspension ou dissous dans un solvant inerte.

5. Procédé selon la revendication 2, caractérisé en ce qu'il est mis en oeuvre à peu près sous la pression atmosphérique.

6. Procédé selon la revendication 2, caractérisé en ce qu'il est mis en oeuvre sous une pression supérieure à la pression atmosphérique.

7. Procédé selon la revendication 1, caractérisé en ce qu'il est mis en oeuvre à une température entre environ 0 et environ 100° C.

8. Procédé selon la revendication 1, caractérisé en ce qu'il est mis en oeuvre à une température inférieure à la température de décomposition du CAN.

9. Procédé selon la revendication 2, caractérisé en ce que l'ammoniac est à l'état liquide.

10. Procédé selon la revendication 1, caractérisé en ce que l'agent réducteur est un alcanol inférieur.

11. Procédé selon la revendication 10, caractérisé en ce que l'alcool est le méthanol.

12. Procédé selon la revendication 10, caractérisé en ce que la réduction est mise en oeuvre en présence d'un catalyseur.

13. Procédé selon la revendication 10, caractérisé en ce que la réaction est mise en oeuvre avec exposition à la lumière ultraviolette.

14. Produit obtenu selon la revendication 1, caractérisé en ce qu'il comprend du nitrate céreux anhydre coordiné avec des ligands de coordination de groupes $NO_3^-$ équilibrés par de ions $NH_4^+$.

15. Produit obtenu selon la revendication 1, caractérisé en ce qu'il consiste en complexe nitrate céreux-nitrate d'ammonium-ammoniac.

16. Complexe nitrate céreux-nitrate d'ammonium-ammoniac anhydre.

17. Complexe selon la revendication 16, caractérisé en ce qu'il répond à la formule (I) :

$Ce(NO_3)_6, 3NH_4NO_3, xNH_3$ (I)

dans laquelle x est supérieur à 0 et inférieur ou égal à 3.

18. Complexe selon la revendication 17, caractérisé en ce qu'il répond à la formule (I), dans laquelle x est compris entre 1 et 3.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | US-A-4 492 655  (GRADEFF) <br> * Colonne 2, lignes 54,55 * <br> --- | 1,2 | C 01 F  17/00 |
| A | INORGANIC CHEMISTRY, vol. 7, no. 4, avril 1968, pages 715-721; T.A. BEINEKE et al.: "The crystal structure of ceric ammonium nitrate" <br> --- | | |
| A | CHEMICAL ABSTRACTS, vol. 77, no. 18, 30 octobre 1972, page 530, résumé no. 121627h; N.B. MIKHEEV et al.: "Reduction of rare earth elements to divalent state by metallic sodium in liquid ammonia", & ZH. NEORG: KHIM. 1972, 17(7), 2052-3 <br> * Résumé * <br> --- | | |
| A | FR-A-2 584 703  (RHONE-POULENC) <br> ----- | | |

**DOMAINES TECHNIQUES RECHERCHES (Int. Cl.4)**

C 01 F  17/00

Le présent rapport a été établi pour toutes les revendications

| Lieu de la recherche | Date d'achèvement de la recherche | Examinateur |
|---|---|---|
| LA HAYE | 20-04-1989 | ZALM W.E. |

EPO FORM 1503 03.82 (P0402)